# EUROPEAN PATENT SPECIFICATION

(11) **EP 1 171 140 B1**
(45) Date of publication and mention of the grant of the patent: **16.06.2004**
(21) Application number: 00922353.8
(22) Date of filing: 19.04.2000
(51) Int. Cl.: A61K 35/14, A61K 41/00, A61P 37/08, A61P 17/00

(54) **TREATMENT OF HYPERSENSITIVITY REACTION DISORDERS**
BEHANDLUNG VON HYPERSENSIBILITÄTREAKTION ERKRANKUNGEN
TRAITEMENT D'AFFECTIONS DUES A DES REACTIONS D'HYPERSENSIBILITE

(30) Priority: 19.04.1999 CA 2269364
(43) Date of publication of application: 16.01.2002
(73) Proprietor: Vasogen Ireland Limited, Shannon, County Clare (IE)
(72) Inventor: SAUDER, Daniel, Toronto, Ontario M4N 3M5 (CA); MANDEL, Arkady Vasogen Inc., Mississauga, Ontario L5L 4M1 (CA); BOLTON, Anthony E., Tideswell, Derbyshire SK17 8HS (GB)
(74) Representative: Gowshall, Jonathan Vallance
(86) International application number: PCT/CA2000/000433
(87) International publication number: WO 2000/062788

(56) References cited:
- WO-A-98/07436
- US-A- 5 147 289
- DATABASE BIOSIS [Online] BIOSCIENCES INFORMATION SERVICE, PHILADELPHIA, PA, US; 1992 VAN IPEREN H P ET AL: "An animal model for extracorporeal photochemotherapy based on contact hypersensitivity." Database accession no. PREV199395005009 XP002152751 & JOURNAL OF PHOTOCHEMISTRY AND PHOTOBIOLOGY B BIOLOGY, vol. 15, no. 4, 1992, pages 361-366, ISSN: 1011-1344
- I. IWAI ET AL.: "UVA- induced immune suppression through an oxidative pathway" THE JOURNAL OF INVESTIGATIVE DERMATOLOGY, vol. 112, no. 1, January 1999 (1999-01), pages 19-24, XP000960536 BALTIMORE, US
- G.M. SHIVJI ET AL.: "Effects of VAS972 therapy on allergic contact hypersensitivity" THE JOURNAL OF INVESTIGATIVE DERMATOLOGY, vol. 114, no. 4, April 2000 (2000-04), page 862 XP000960563 BALTIMORE, US
- G.M. SHIVJI ET AL.: "The effect of VAS972 on allergic contact hypersensitivity" JOURNAL OF CUTANEOUS MEDICINE AND SURGERY, vol. 4, no. 3, July 2000 (2000-07), pages 132-137, XP000960566

## Description

### FIELD OF THE INVENTION

This invention relates to the field of medicine and medical treatments. In particular, the invention relates to the use of treated, modified mammalian blood for treatment and prophylaxis of T-cell mediated delayed type hypersensitivity reactions in mammalian patients.

### BACKGROUND OF THE INVENTION

T-cell mediated delayed type hypersensitivity reactions as the term is used herein means adverse reactions on the part of a mammalian patient to foreign agents, commonly but not exclusively manifested by development of skin disorders on the patient, and in which the disorder takes at least 24 hours to exhibit full manifestation. Many of these are diagnosable by skin tests. The reactions may be chemical contact reactions, food ingestion reactions or drug ingestion reactions. Specific examples of such conditions include contact hypersensitivity reactions, in which the skin of the patient exhibits a reaction to an agent which the body has previously encountered, by contact or by inoculation. The "poison ivy" type of reaction is a specific example of contact hypersensitivity. Hypersensitivity to β-lactam antibiotics (e.g. penicillins) is an example where inoculation of a foreign agent gives rise to a skin disorder-manifested, T-cell mediated delayed type hypersensitivity. The external agents can be plant, animal, insect or reptilian secretions, chemical or biochemical antigens, from synthetic or natural sources. Various types of fibers, fabrics and the like, such as latex used in surgical gloves, can give rise to T-cell mediated hypersensitivity reaction in certain individuals. The offending external agents can be water-borne agents such as dissolved salts and minerals, encountered for example in environmental, mining, metallurgical and chemical manufacturing operations. T-cell mediated delayed type hypersensitivity reactions are to be distinguished from psoriasis, which is an autoimmune disorder which manifests itself in red scaly skin patches having an inflammatory component, but not resulting from contact reaction.

Mammalian blood modified by exposure simultaneously to certain stressors has been reported to be useful for the treatment of a variety of pathological conditions. The stressors to which the blood is exposed are an oxidative environment namely ozone/oxygen gas mixtures applied to the blood, a temperature stressor and UV light. Thus:
U.S. Patent No. 4,968,483 Mueller et al. describes an apparatus for oxygenating blood by treating an aliquot of a patient's blood extracorporeally, with an oxygen/ozone mixture and ultraviolet light, at a controlled temperature. The apparatus taught by Mueller is proposed for use in hematological oxidation therapy.
U.S. Patent No. 5,591,457 Bolton discloses a method of inhibiting the aggregation of blood platelets in a human, a method of stimulating the immune system and a method of treating peripheral vascular diseases such as Raynaud's disease, by extracting an aliquot of blood from a patient, subjecting it to an ozone/oxygen gas mixture and ultraviolet radiation at a temperature in the range of about 37 to 43°C, and then re-injecting the treated blood in the human patient.

International Patent Application PCT/GB93/00259 Bolton describes a process for increasing the content of nitric oxide in the blood of a mammalian subject, potentially useful in treating conditions such as high blood pressure in mammalian subjects, by subjecting a sample of the patient's blood extracorporeally to three stressors simultaneously, namely an ozone/oxygen gas mixture bubbled through the blood sample, exposure to UV radiation and an elevated temperature, followed by re-injection of the treated blood sample into the patient.

International Publication No. WO 98/07436 describes an autoimmune vaccine for administration to human patients to alleviate the symptoms of autoimmune diseases such as rheumatoid arthritis. The vaccine comprises an aliquot of the subject's blood which has been subjected extracorporeally to an oxidizing environment, UV radiation and elevated temperature, simultaneously.

International Publication No. WO 96/34613 relates to treatment of vascular disorders associated with deficient endothelial function, in a mammalian subject, by administration to the patient of an aliquot of blood which has been modified by having been subjected simultaneously to stressors namely elevated temperature in the range of 37° to 55°C, ultraviolet radiation and an oxidative environment

### SUMMARY OF THE INVENTION

According to one aspect, the present invention provides use for treatment or prophylaxis of T-cell mediated delayed type hypersensitivity disorders in a mammalian patient, of modified mammalian blood having been modified extracorporeally by simultaneous or sequential exposure to the stress of an oxidative environment and the stress of UV radiation.

### BRIEF DESCRIPTION OF THE DRAWINGS

The accompanying FIGURES are presentations of the results of specific Examples described below.

### DETAILED DESCRIPTION OF PREFERRED EMBODIMENTS

According to a preferred use of the present invention, an aliquot of blood is extracted from a mammalian subject, preferably a human, and the aliquot of blood is treated ex vivo, simultaneously or sequentially, with the aforementioned stressors. Then it is to be injected back into the same subject. Preferably a combination of both of the aforementioned stressors is used.

Preferably also, the aliquot of blood is in addition subjected to mechanical stress. Such mechanical stress is suitably that applied to the aliquot of blood by extraction of the blood aliquot through a conventional blood extraction needle, or a substantially equivalent mechanical stress, applied shortly before the other chosen stressors are applied to the blood aliquot. This mechanical stress may be supplemented by the mechanical stress exerted on the blood aliquot by bubbling gases through it, such as ozone/oxygen mixtures, as described below. Optionally also, a temperature stressor may be applied to the blood aliquot, simultaneously or sequentially with the other stressors, i.e. a temperature at, above or below body temperature.

The terms "aliquot", "aliquot of blood" or similar terms used herein include whole blood, separated cellular fractions of the blood including platelets, separated non-cellular fractions of the blood including plasma, plasma components and combinations thereof. Preferably, in human patients, the volume of the aliquot is up to about 400 ml, preferably from about 0.1 to about 100 ml, more preferably from about 1 to about 15 ml, even more preferably from about 8 to about 12 ml, and most preferably about 10 ml. The effect of the combination of stressors is to modify the blood, and/or the cellular or non-cellular fractions thereof, contained in the aliquot. The modified aliquot is then to be re-introduced into the subject's body by any suitable means, most preferably intramuscular injection, but also including subcutaneous injection, intraperitoneal injection, intra-arterial injection, intravenous injection and oral administration.

The optionally applied temperature stressor either warms the aliquot being treated to a temperature above normal body temperature or cools the aliquot below normal body temperature. The temperature is selected so that the temperature stressor does not cause excessive hemolysis in the blood contained in the aliquot and so that, when the treated aliquot is injected into a subject, the desired effect will be achieved. Preferably, the temperature stressor is applied so that the temperature of all or a part of the aliquot is up to about 55°C, and more preferably in the range of from about -5°C to about 55°C.

In some preferred embodiments of the invention, the temperature of the aliquot is raised above normal body temperature, such that the mean temperature of the aliquot does not exceed a temperature of about 55°C i.e., the temperature stressor is in the range of 37-55°C, more preferably from about 40°C to about 50°C, even more preferably from about 40°C to about 44°C, and most preferably about 42.5 ± 1 °C.

In other preferred embodiments, the aliquot is cooled below normal body temperature such that the mean temperature of the aliquot is within the range of from about 4°C to about 36.5°C, more preferably from about 10°C to about 30°C, and even more preferably from about 15°C to about 25°C

The oxidative environment stressor can be the application to the aliquot of solid, liquid or gaseous oxidizing agents. Preferably, it involves exposing the aliquot to a mixture of medical grade oxygen and ozone gas, most preferably by applying to the aliquot medical grade oxygen gas having ozone as a component therein. The ozone content of the gas stream and the flow rate of the gas stream are preferably selected such that the amount of ozone introduced to the blood aliquot, either on its own or in combination with one of the other stressors, does not give rise to excessive levels of cell damage, and so that, when the treated aliquot is injected into a subject, the desired effect will be achieved. Suitably, the gas stream has an ozone content of up to about 300 µg/ml, preferably up to about 100 µg/ml, more preferably about 30 µg/ml, even more preferably up to about 20 µg/ml, particularly preferably from about 10 µg/ml to about 20 µg/ml, and most preferably about 14.5 ± 1.0µ g/ml. The gas stream is suitably supplied to the aliquot at a rate of up to about 2.0 litres/min, preferably up to about 0.5 litres/min, more preferably up to about 0.4 litres/min, even more preferably up to about 0.33 litres/min, and most preferably about 0.24 ± 0.024 litres/min. The lower limit of the flow rate of the gas stream is preferably not lower than 0.01 litres/min, more preferably not lower than 0.1 litres/min, and even more preferably not lower than 0.2 litres/min.

The ultraviolet light stressor is suitably applied by irradiating the aliquot under treatment from a source of UV light. Preferred UV sources are UV lamps emitting UV-C band wavelengths, i.e. at wavelengths shorter than about 280 nm. Ultraviolet light corresponding to standard UV-A (wavelengths from about 315 to about 400 nm) and UV-B (wavelengths from about 280 to about 315) sources can also be used. As in the case of the oxidative stressor, the UV dose should be selected, on its own or in combination of the other chosen stressor(s), so that excessive amounts of cell damage do not occur, and so that, when the treated aliquot is injected into a subject, the desired effect will be achieved. For example, an appropriate dosage of such UV light, can be obtained from up to eight lamps arranged to be exposed to the sample container holding the aliquot, operated at an intensity to deliver a total UV light energy at 253.7 nm at the surface of the blood of from about 0.025 to about 10 joules/cm², preferably from about 0.1 to about 3.0 joules/cm². Such a treatment, applied in combination with the oxidative environment stressor, provides a modified blood aliquot which is ready for injection into the subject.

It is preferred to subject the aliquot to the oxidative environment stressor, the UV light stressor and the temperature stressor simultaneously, following the subjection of the aliquot to the mechanical stress, e.g. by extraction of the blood from the patient. Thus, the aliquot may be maintained at a predetermined temperature above or below body temperature while the oxygen/ozone gas mixture is applied thereto and while it is irradiated with ultraviolet light.

The time for which the aliquot is subjected to the stressors is normally within the time range of from about 0.5 minutes up to about 60 minutes. The time depends to some extent upon the chosen combination of stressors. When UV light is used, the intensity of the UV light may affect the preferred time. The chosen temperature level may also affect the preferred time. When oxidative environment in the form of a gaseous mixture of oxygen and ozone applied to the aliquot is chosen as one of the two stressors, the concentration of the oxidizing agent and the rate at which it is supplied to the aliquot may affect the preferred temperature. Some experimentation to establish optimum times may be necessary on the part of the operator, once the other stressor levels have been set. Under most stressor conditions, preferred times will be in the approximate range of from about 2 to about 5 minutes, more preferably about 3 minutes. The starting blood temperature, and the rate at which it can be warmed or cooled to a predetermined temperature, tends to vary from subject to subject. Warming is suitably by use of one or more infrared lamps placed adjacent to the aliquot container. Other methods of warming can also be adopted.

As noted, it is preferred to subject the aliquot of blood to a mechanical stressor, as well as the chosen stressor(s) discussed above. Extraction of the blood aliquot from the patient through an injection needle constitutes the most convenient way of obtaining the aliquot for further extracorporeal treatment, and this extraction procedure imparts a suitable mechanical stress to the blood aliquot. The mechanical stressor may be supplemented by subsequent processing, for example the additional mechanical shear stress caused by bubbling as the oxidative stressor is applied.

In the practice of the present invention, the blood aliquot may be treated with the heat, UV light and oxidative environment stressors using an apparatus of the type described in aforementioned U.S. Patent No. 4,968,483 to Mueller. The aliquot is placed in a suitable, sterile container, which is fitted into the machine. A UV-permeable container is used and the UV lamps are switched on for a fixed period before the other stressor is applied, to allow the output of the UV lamps to stabilize. When a temperature stressor is used combination, the UV lamps are typically on while the temperature of the aliquot is adjusted to the predetermined value, e.g. 42.5 ± 1 °C. Four UV lamps are suitably used, placed around the container.

Preferably, a mammalian patient is given one or more courses of treatments, each course of treatment comprising the administration to a mammalian subject of one or more (e.g. one to six) aliquots of mammalian blood modified as discussed above.

For optimum effectiveness of the treatment, it is preferred that no more than one aliquot of modified blood be administered to the subject per day, in one or more injection sites, and that the maximum rest period between any two consecutive aliquots during the course of treatment be no greater than about 21 days. As used herein, the term "rest period" is defined as the number of days between consecutive aliquots or consecutive courses of treatment on which no aliquots of modified blood are administered to the subject.

Therefore, except where aliquots are administered to the subject on consecutive days, a rest period of from 1 to 21 days is provided between any two aliquots during the course of treatment. Moreover, at least one of the rest periods during the course of treatment preferably has a length of about 3 to 15 days.

Although it may be sufficient to administer only one course of treatment as described above to the subject, it may be preferred in some circumstances to administer more than one course of treatment, or to follow the above-described course of treatment by periodic "booster" treatments, if necessary, to maintain the desired effects. For example, it may be preferred to administer booster treatments at intervals of 3 to 4 months following the initial course of treatment, or to administer a second course of treatments to the subject following a rest period of several weeks or months.

The use according to the present invention shows potential in the treatment and prophylaxis of a wide variety of T-cell mediated delayed type hypersensitivity reactions, including those mentioned above. In particular, the following conditions show particularly attractive potential for treatment with the process of the invention:
contact hypersensitivity to plant and animal secretions such as poison ivy, poison oak and nettles (urticaria);
eczema;
atopic dermatitis;
erythema multiforma;
angioedema vasculitis;
atopic conjunctivitis;
skin reactions to contact with certain chemicals e.g. nickel, latex, etc., in solid or solution form;
reactions to drug administrations, especially β-lactam antibiotic administration;
protein-induced reactions to food ingestion.

The invention is further illustrated and described with reference to the following specific examples, comprising animal studies conducted in an approved manner.

### EXAMPLE 1

The effectiveness of the treatment according to a preferred embodiment of the present invention, on contact hypersensitivity (CHS), was assessed on laboratory mice, according to approved animal experimentation procedures, using the method described by Kondo et. al., "Lymphocyte function associated antigen-1 (LFA-1) is required for maximum elicitation of allergic contact dematitis" Br J.Dermatol. 131:354-359, 1994, with minor variations. Briefly, to induce CHS, the abdominal skin of each mouse was shaved and painted with dinitrodifluorobenzene DNFB, the sensitizing chemical, using 25 µl of 0.5% DNFB in 4:1 acetone:olive oil solution. This sensitization was applied to four groups of five Balb C mice.

Whole blood was obtained from Balb C mice, by extraction from a main artery through an injection needle, and treated with an anti-coagulant. An aliquot of this was subjected to the process of a preferred embodiment of the invention, to obtain treated blood. The remainder was left untreated, for use in control experiments. Since these mice are genetically identical, the administration of the treated blood to others of the group is equivalent to administration of the treated blood to the donor animal.

To obtain treated blood, the selected aliquot, in a sterile, UV-transmissive container, was treated simultaneously with a gaseous oxygen/ozone mixture and ultraviolet light at elevated temperature using an apparatus as generally described in aforementioned U.S.Patent No. 4,968,483 Mueller et.al. Specifically, 10 ml of citrated blood was transferred to a sterile, low density polyethylene vessel (more specifically, a Vasogen VC7002 Blood Container) for ex vivo treatment with stressors according to the invention. Using an apparatus as described in the aforementioned Mueller patent (more specifically, a Vasogen VC7001 apparatus), the blood was heated to 42.5± 1 °C and at that temperature irradiated with UV light principally at a wavelength of 253.7 nm, while oxygen/ozone gas mixture was bubbled through the blood to provide the oxidative environment and to facilitate exposure of the blood to UV. The constitution of the gas mixture was 14.5 ± 1.0 µg ozone/ml, with the remainder of the mixture comprising medical grade oxygen. The gas mixture was bubbled through the aliquot at a rate of 240 ± 24 ml/min for a period of 3 minutes.

Of the 4 groups of sensitized mice, the first, control group A-1 received no treatment. The second, control group B-1, was treated with physiological saline, 50µl. The third, control group C-1, was sham treated, with 50µl of blood which had been extracted but not treated with the stressors. The fourth, test group D-1, was treated with 50µl of blood subjected to stressors as described above. Treatments, each involving intramuscular injection of 50 µl of the respective liquid, started on the day of sensitization, and was repeated every day for a total of 6 days. On the same day as the last treatment, but after its administration, the animals were challenged with DNFB, by applying to the ears of each animal 10µl of 0.2% solution of DNFB. Inflammation due to CHS manifests itself in a swelling of the ears. Ear thickness was measured, 24 hours after challenge, with a Peacock spring-loaded micrometer (Ozaki Co., Tokyo, Japan). The results were expressed as the change (from pre-challenge level) in ear thickness and represent the mean maximal increase at 24 hours after challenge.

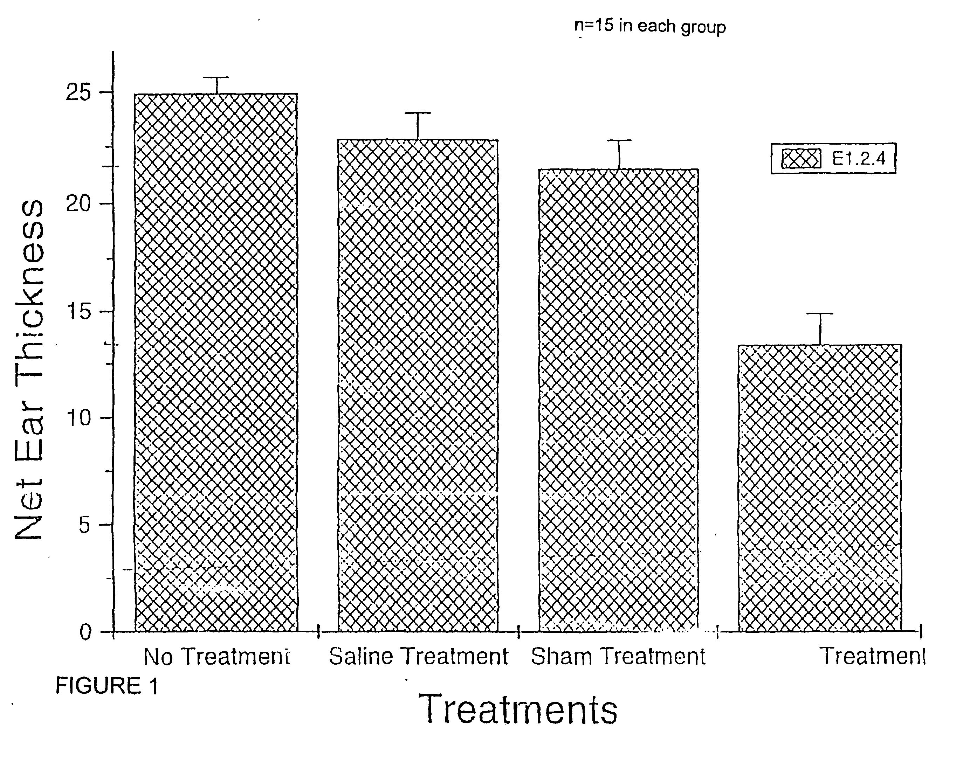

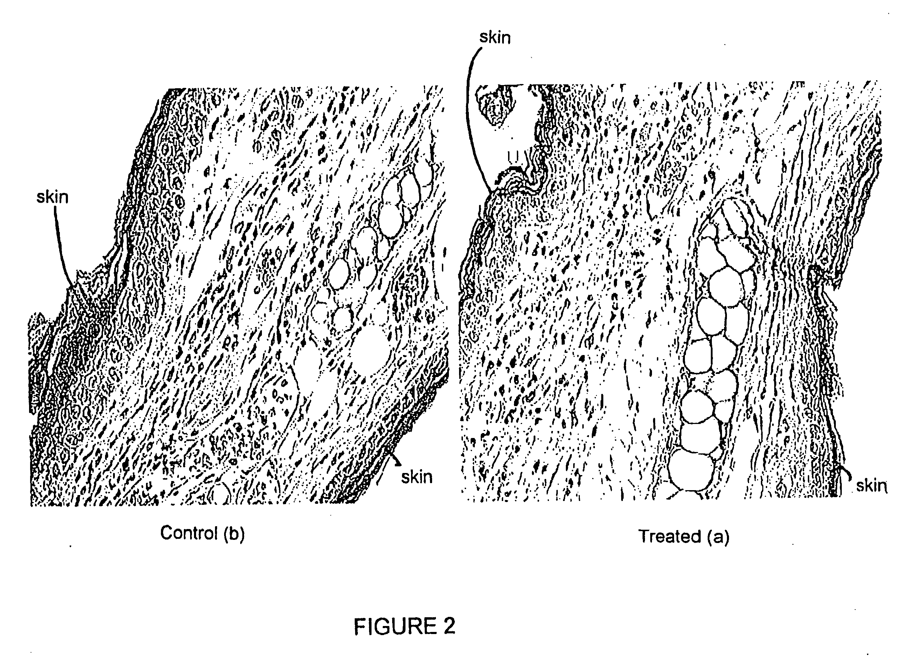

The experiments were repeated two more times, using two more sets of four groups of animals, to ensure statistical significance in the results. Figure 1 of the accompanying drawings is a graphical presentation of these results. A notable and significant reduction in ear thickness (inflammation) is to be observed with the animals treated according to this preferred process of the invention, as compared with any of the other groups. Figure 2 of the accompanying drawings represent photographs of cross-sections of the ears of a representative treated animal of group D-1 (picture (a)) and a representative untreated group A-1 animal (picture(b)). The decreased skin thickness, and the reduced lymphocyte infiltration (lower density of dark stained cells) is readily apparent on picture (a) from the treated animal, further demonstrating a significant reduction in inflammation.

The percentage suppression when compared with the standard CHS response (no treatment, control group A-1) is 8% for the saline treatment group B-1, 14% for the sham treatment group C-1 and 46% for group D-1, treated according to the embodiment of the invention.

### EXAMPLE 2

The procedure of Example was followed, using four groups of Balb/C mice, with one group receiving a blood aliquot which had been subjected to UV and ozone/oxygen bubbling, as described, but without application of the heat stressor (i.e. treated at room temperature). Thus, group A-2 received no treatment, group B-2 received untreated blood (sham treatment), group C-2 received blood treated with UV and ozone but no heat, and group D-2 received blood treated the same way as in the case of group D-1 of Example 1.

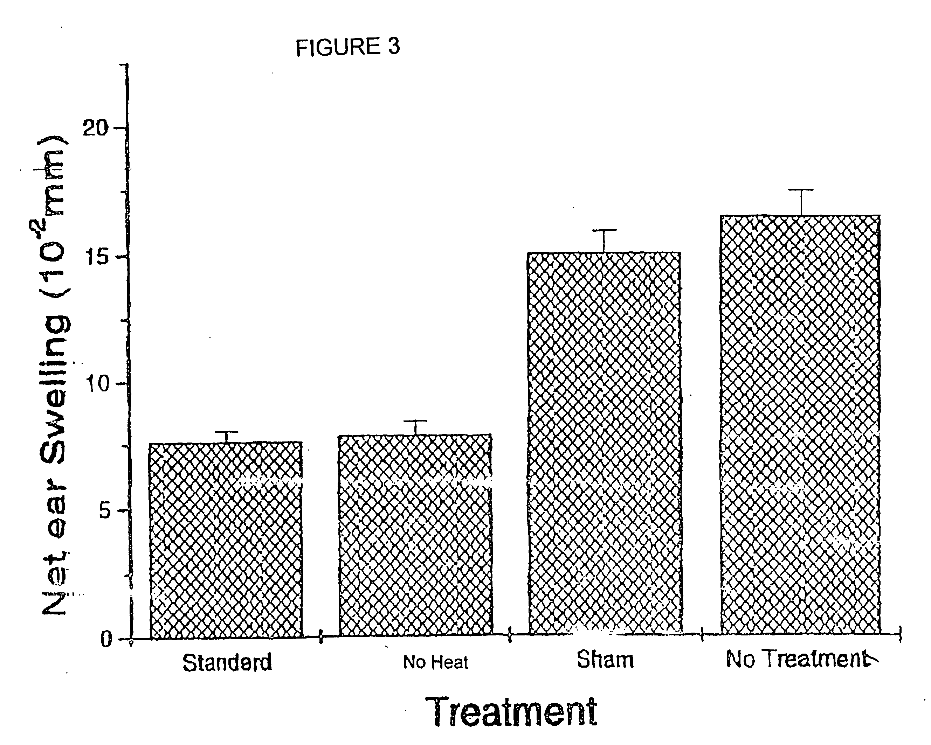

The results are presented graphically on Fig. 3, in the same manner as Fig. 1. The result from group D-2 is marginally better than that from group C-2. The percentage suppression when compared to the standard CHS response (no treatment, group A-2) is 9% for group B-2, sham treatment, 52.5% for group C-2 and 54% for group D-2.

### EXAMPLE 3

Whole blood was obtained from Balb/C mice. Part of the blood was subjected to UV, ozone and heat treatment as described in Example 1, and part of the blood remained untreated. Both the untreated blood and the treated blood were centrifuged to obtain a cellular fraction, and washed with saline. The treated and untreated fractions were administered to animals challenged with DNFB to develop contact hypersensitivity as described in Example 1.

Four groups of 5 mice each were injected according to the schedule of Example 1, and evaluated, as follows: Group A-3 - no-treatment; Group B-3 - cellular fraction of sham treated blood; Group C-3 - cellular part of treated blood; Group D-3 - whole treated blood. The administrations to the mice took place just prior to sensitization with 0.5% DNFB and continued every day until challenge with 0.2% DNFB, 5 days later. A total of 6 injections were administered to each mouse.

The ear swelling of each mouse was measured 24 hours after challenge. Each experiment was repeated three times, to ensure statistical significance of the results. Net ear swelling as a measure of contact hypersensitivity and suppression thereof was calculated as 1 - (ear swelling of blood administer mouse/ ear swelling of no blood administered mouse) x 100.

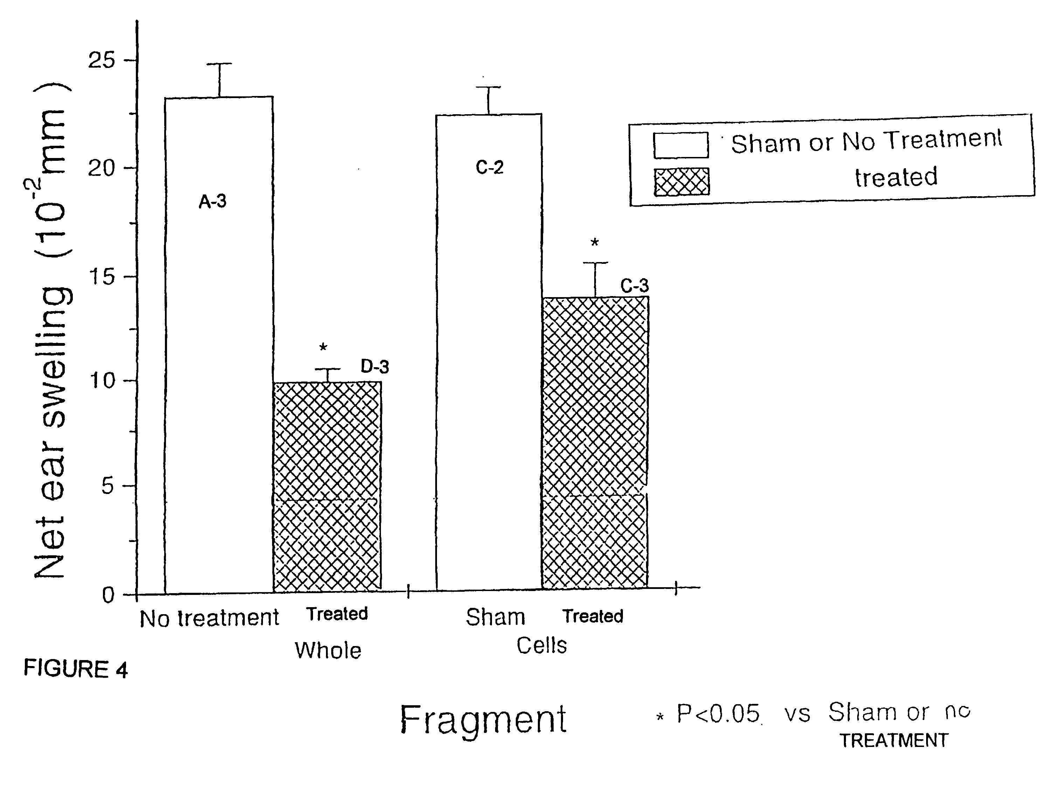

The results are presented graphically on Fig. 4., a summary of three experiments. A significant suppression of CHS is seen with the cellular fraction of the treated blood. There was no significant difference between the treated cellular fraction and treated whole blood.

## Claims

1. Use of an aliquot of modified mammalian blood, the blood aliquot having been modified extra corporeally by simultaneous or sequential exposure to the stress of an oxidative environment and the stress of UV radiation for the manufacture of a medicament for the treatment or prophylaxis of T-cell mediated delayed type hypersensitivity disorders in a mammalian patient by administration to the patient.

2. Use according to Claim 1 wherein the stressors have been applied simultaneously to the blood aliquot.

3. Use according to Claim 1 or Claim 2, wherein mechanical stress has been also applied to the aliquot.

4. Use according to Claim 1, Claim 2 or Claim 3 wherein the blood aliquot has been further subjected to a temperature stress, simultaneously with the application of the UV stress and the oxidative environment stress.

5. Use according to any preceding claim wherein the oxidative environment is a gaseous mixture of oxygen and ozone, bubbled through the aliquot.

6. Use according to Claim 4 or Claim 5 as appendant to Claim 4 wherein the temperature stressor is a temperature in the approximate range 37-55°C.

7. Use according to any preceding claim wherein the aliquot of blood has a volume of from 0.1 - 100 ml.

8. Use according to any preceding claim wherein the oxidative environment is a gas stream of ozone and medical grade oxygen, bubbled through the aliquot, the stream having an ozone content of up to 300 µg per ml, supplied to the aliquot at a rate of up to about 2.0 litres per minute.

9. Use according to any preceding claim wherein the UV stressor is UV light of wavelength shorter than about 280 nm.

10. Use according to any preceding claim wherein the aliquot has been subjected to the UV stressor and the oxidative environment stressor for a period of time from about 0.5 to 60 minutes.

11. Use according to any preceding claim where the disorder is a contact hypersensitivity.

12. Use according to Claim 11 wherein the contact hypersensitivity is a hypersensitivity to a plant secretion.

13. Use according to any of Claims 1-10 wherein the disorder is selected from eczema, atopic dermatitis, erythema multiforma, angioedema vasculitis, and atopic conjunctivitis.

14. Use according to any of Claims 1-10 wherein the disorder is contact hypersensitivity skin reaction to chemical solids or solutions.

15. Use according to any of Claims 1-10 wherein the disorder is a reaction to β-lactam antibiotic administration.

16. Use according to any of Claims 1-10 wherein the disorder is a protein induced reaction to food ingestion.

## Patentansprüche

1. Verwendung eines Aliquots von modifiziertem Säugerblut, wobei das Säugerblut extrakorporal durch gleichzeitige oder aufeinanderfolgende Einwirkung des Stresses einer oxidativen Umgebung und des Stresses von UV-Bestrahlung modifiziert worden ist, zur Herstellung eines Arzneimittels zur Behandlung oder Prophylaxe von T-Zell-vermittelten verzögerten Hypersensibilitätserkrankungen in einem Säugerpatienten.

2. Verwendung nach Anspruch 1, **dadurch gekennzeichnet, daß** die Stressoren gleichzeitig auf das Blutaliquot angewendet worden sind.

3. Verwendung nach Anspruch 1 oder Anspruch 2, **dadurch gekennzeichnet, daß** mechanischer Streß ebenfalls auf das Aliquot angewendet worden ist

4. Verwendung nach Anspruch 1, Anspruch 2 oder Anspruch 3, **dadurch gekennzeichnet, daß** das Blutaliquot außerdem einem Temperaturstreß unterworfen worden ist, gleichzeitig mit der Anwendung des UV-Stresses und des Stresses einer oxidativen Umgebung.

5. Verwendung nach einem vorangehenden Anspruch, **dadurch gekennzeichnet, daß** die oxidative Umgebung eine gasförmige Mischung aus Sauerstoff und Ozon ist, die durch das Aliquot hindurchgeleitet wird.

6. Verwendung nach Anspruch 4 oder Anspruch 5, sofern abhängig von Anspruch 4, **dadurch gekennzeichnet, daß** der Temperaturstressor eine Temperatur im ungefähren Bereich 37 bis 55°C ist.

7. Verwendung nach einem vorangehenden Anspruch, **dadurch gekennzeichnet, daß** das Blutaliquot ein Volumen von 0,1 bis 100 ml besitzt.

8. Verwendung nach einem vorangehenden Anspruch, **dadurch gekennzeichnet, daß** die oxidative Umgebung ein Gasstrom aus Ozon und Sauerstoff medizinischer Qualität ist, der durch das Aliquot hindurchgeleitet wird, wobei der Strom einen Ozongehalt von bis zu 300 µg pro ml besitzt, zugeführt zum Aliquot mit einer Geschwindigkeit von bis zu 2,0 Litern pro Minute.

9. Verwendung nach einem vorangehenden Anspruch, **dadurch gekennzeichnet, daß** der UV-Stressor UV-Licht mit einer kürzeren Wellenlänge als etwa 280 nm ist.

10. Verwendung nach einem vorangehenden Anspruch, **dadurch gekennzeichnet, daß** das Aliquot dem UV-Stressor und dem Stressor einer oxidativen Umgebung für einen Zeitraum von etwa 0,5 bis etwa 60 Minuten unterworfen worden ist.

11. Verwendung nach einem vorangehenden Anspruch, **dadurch gekennzeichnet, daß** die Erkrankung eine Kontakthypersensibilität ist.

12. Verwendung nach Anspruch 11, **dadurch gekennzeichnet, daß** die Kontakthypersensibilität eine Hypersensibilität gegenüber einer Pflanzensekretion ist.

13. Verwendung nach einem der Ansprüche 1 bis 10, **dadurch gekennzeichnet, daß** die Erkrankung ausgewählt ist aus Ekzem, atopischer Dermatitis, Erythema multiforma, Quinckeschem Ödem und atopischer Konjunktivitis.

14. Verwendung nach einem der Ansprüche 1 bis 10, **dadurch gekennzeichnet, daß** die Erkrankung Kontakthypersensibilitätshautreaktion gegenüber chemischen Feststoffen oder Lösungen ist.

15. Verwendung nach einem der Ansprüche 1 bis 10, **dadurch gekennzeichnet, daß** die Erkrankung eine Reaktion auf die Verabreichung eines β-Lactam-Antibiotikums ist.

16. Verwendung nach einem der Ansprüche 1 bis 10, **dadurch gekennzeichnet, daß** die Erkrankung eine Protein-induzierte Reaktion auf Nahrungsaufnahme ist.

## Revendications

1. Utilisation d'une aliquote de sang modifié d'origine mammalienne, l'aliquote de sang ayant été modifiée extra-corporellement en la soumettant, de façon simultanée ou séquentielle, à l'agression d'un environnement oxydatif et à l'agression d'un rayonnement ultraviolet (UV), en vue de fabriquer un médicament destiné au traitement ou à la prévention des troubles d'hypersensibilité retardée médiée par cellules T, chez un patient mammalien :

2. Utilisation conforme à la revendication 1, dans laquelle les agents d'agression ont été appliqués à l'aliquote de sang de façon simultanée.

3. Utilisation conforme à la revendication 1 ou à la revendication 2, dans laquelle a été également appliquée à l'aliquote une agression mécanique.

4. Utilisation conforme à la revendication 1, à la revendication 2 ou à la revendication 3, dans laquelle l'aliquote de sang a été en outre soumise à une agression thermique simultanément à l'application d'une agression par rayonnement UV et d'une agression par environnement oxydatif.

5. Utilisation conforme à l'une des revendications précédentes dans laquelle l'environnement oxydatif est un mélange gazeux, composé d'oxygène et d'ozone, que l'on fait barboter à travers l'aliquote.

6. Utilisation conforme à la revendication 4 ou à la revendication 5 telle que jointe à la revendication 4 dans laquelle l'agent d'agression thermique est une température approximativement comprise entre 37 °C et 55 °C.

7. Utilisation conforme à l'une des revendications précédentes dans laquelle l'aliquote de sang a un volume de 0,1 à 100 mL.

8. Utilisation conforme à l'une des revendications précédentes dans laquelle l'environnement oxydatif est un courant gazeux, composé d'ozone et d'oxygène de grade médical, que l'on fait barboter à travers l'aliquote, le courant ayant un taux d'ozone au plus de 300 µg par millilitre et un débit dans l'aliquote au plus d'environ 2,0 litres par minute.

9. Utilisation conforme à l'une des revendications précédentes dans laquelle l'agent d'agression par rayonnement UV est une lumière UV de longueur d'onde inférieure à environ 280 nm.

10. Utilisation conforme à l'une des revendications précédentes dans laquelle l'aliquote a été soumise à l'agent d'agression par rayonnement UV et à l'agent d'agression par environnement oxydatif pendant une durée approximativement comprise entre 0,5 et 60 minutes.

11. Utilisation conforme à l'une des revendications précédentes dans laquelle le trouble est une hypersensibilité de contact.

12. Utilisation conforme à la revendication 11 dans laquelle l'hypersensibilité de contact est une hypersensibilité à une sécrétion végétale.

13. Utilisation conforme à l'une des revendications 1 à 10 dans laquelle le trouble appartient à l'ensemble constitué par l'eczéma, la dermatite atopique, l'érythème polymorphe, la vascularite angio-oedème, et la conjonctivite atopique.

14. Utilisation conforme à l'une des revendications 1 à 10 dans laquelle le trouble est une réaction dermique d'hypersensibilité de contact à des composés chimiques solides ou en solution.

15. Utilisation conforme à l'une des revendications 1 à 10 dans laquelle le trouble est une réaction à l'administration d'antibiotiques de type β-lactames.

16. Utilisation conforme à l'une des revendications 1 à 10 dans laquelle le trouble est une réaction induite par une protéine à une ingestion alimentaire.
